# EUROPÄISCHE PATENTSCHRIFT

(11) **EP 1 214 345 B1**
(45) Veröffentlichungstag und Bekanntmachung des Hinweises auf die Patenterteilung: **29.12.2004**
(21) Anmeldenummer: 00972578.9
(22) Anmeldetag: 07.09.2000
(51) Int. Cl.: C07K 14/47

(54) **MITTEL ZUR THERAPIE VON MENSCHLICHEN ERKRANKUNGEN, INSBESONDERE FÜR DIE THERAPIE VON TUMOREN WIE KOLONKARZINOMEN UND MELANOMEN ODER ZUR GEWEBEREGENERATION UND FÖRDERUNG DES HAARWUCHSES**
AGENTS FOR TREATING HUMAN DISEASES, ESPECIALLY FOR TREATING TUMORS SUCH AS COLONIC CANCERS AND MELANOMAS OR FOR REGENERATING TISSUE AND PROMOTING HAIR GROWTH
AGENTS SERVANT A TRAITER DES MALADIES HUMAINES, NOTAMMENT DES TUMEURS TELLES QUE LES CARCINOMES DU COLON ET LES MELANOMES, OU SERVANT A REGENERER DES TISSUS ET A FAVORISER LA CROISSANCE DES CHEVEUX

(30) Priorität: 16.09.1999 DE 19944404
(43) Veröffentlichungstag der Anmeldung: 19.06.2002
(73) Patentinhaber: MAX-DELBRÜCK-CENTRUM FÜR MOLEKULARE MEDIZIN, 13125 Berlin (DE)
(72) Erfinder: BIRCHMEIER, Walter, 16341 Schwanebeck (DE); VON KRIES, Jens-Peter, 16341 Zepernick (DE)
(74) Vertreter: Krauss, Jan B., Dr.
(86) Internationale Anmeldenummer: PCT/DE2000/003104
(87) Internationale Veröffentlichungsnummer: WO 2001/019353

(56) Entgegenhaltungen:
- WO-A-98/42296
- WO-A-99/42481
- MORIN P J ET AL: "Activation of beta-catenin-Tcf signaling in colon cancer by mutations in beta-catenin or APC" SCIENCE, AMERICAN ASSOCIATION FOR THE ADVANCEMENT OF SCIENCE,, US, Bd. 275, 21. März 1997 (1997-03-21), Seiten 1787-1790, XP002088507 ISSN: 0036-8075

## Beschreibung

Die Erfindung betrifft ein Verfahren zur Auffindung von Substanzen als Mittel zur Therapie von menschlichen Erkrankungen auf der Basis von Substanzen, die die Bindung von β-Catenin an LEF-1/TCF-Transkriptionsfaktoren, APC oder Conductin/Axin verhindern. Bevorzugt sind diese Mittel zur Therapie von Tumoren wie Kolonkarzinomen und Melanomen oder zur Geweberegeneration und Förderung des Haarwuchses einsetzbar. Anwendungsgebiete der Erfindung sind die Medizin und die pharmazeutische Industrie.

β-Catenin ist ein zytoplasmatisches Protein mit verschiedenen Funktionen in der Zelle. Im Komplex mit den Zelladhäsionsmotekülen der Cadherin-Familie stellt β-Catenin die Verbindung zum Zytoskelett her (Hülsken, J. et al., E-cadherin and APC compete for the interaction with beta-catenin and the cytoskeleton. J-Cell-Biol. 127: 2061-9, 1994). Zusätzlich ist β-Catenin eine Komponente der Wnt-Signaltransduktion, die in der Embryonalentwicklung eine große Rolle spielt. Der Transkriptionsfaktor LEF-1 wurde als Interaktionspartner von β-Catenin in dieser Signalkaskade indentifiziert (Behrens, J. et al., Functional interaction of beta-catenin with the transcription factor LEF-1. Nature, 382: 638-42, 1996). Der Mechanismus der Signaltransduktion durch β-Catenin und LEF-1 ist geklärt: Er besteht in dem durch LEF-1 vermittelten Transport von β-Catenin in den Zellkern. Im Zellkern reguliert dieser Komplex die Genexpression durch die im Komplexveränderte, LEF-1 induzierte DNA-Biegung und durch die carboxy-terminale Transaktivierungsdomäne von β-Catenin. Inzwischen wurde gezeigt, daß auch andere Mitglieder der LEF-1/TCF-Familie von Transkriptionsfaktoren, z.B. TCF-4 diese Signaltransduktion vermitteln können (Korinek, V. et al., Constitutive transcriptional activation by a beta-catenin-Tcf complex in APC-/- colon carcinoma. Science, 275: 1784-87,1997).

Voraussetzung für diese β-Catenin-abhängige Signaltransduktion ist die Stabilisierung des zytoplasmatischen Pools von freiem, nicht Cadherin-gebundenem β-Catenin. Dieser Pool wird durch die Glykogen-Synthetase-Kinase 3β, durch das Tumorsuppressor-Genprodukt APC sowie durch Conductin/Axin negativ reguliert.

Für Karzinome und Melanome wurde gezeigt, daß Mutationen im N-Terminus von β-Catenin oder in der β-Catenin-Bindungsdomäne von APC diese Regulation aufheben (Morin, P.J. et al., Activation of beta-catenin-Tcf signaling in colon cancer by mutations in beta-catenin or APC. Science, 275: 1787-90, 1997). Als Konsequenz wird der β-Catenin-Pool stabilisiert. In Melanomen führt diese Stabilisierung zur LEF-1 vermittelten Translokation von β-Catenin in den Zellkern, während in Kolonkarzinomen vor allemTCF-4 diese Funktion erfüllt. Die transkriptionelle Aktivität des Komplexes in Karzinom-Zellinien wurde durch die Aktivierung eines Reportergens belegt. Darüber hinaus konnte gezeigt werden, daß diese Aktivität in APC-defizienten Kolonkarzinom-Zellinien nach Wiedereinführung von APC inhibiert wurde.

APC-Mutationen wurden in der überwiegenden Mehrheit von Kolonkarzinomen identifiziert, während nicht APC-defiziente Tumore Mutationen im β-Catenin-Gen aufweisen. Das Resultat dieser Mutationen von APC oder β-Catenin ist die Aktivierung der Signaltransduktion durch den β-Catenin-LEF/TCF-Komplex. Es unterstreicht die Schlüsselrolle von β-Catenin in der Tumorentstehung. Da APC-Mutationen als ein frühes Ereignis in der Entstehung von Kolontumoren identifiziert wurden, ist die Aktivierung des β-Catenin-LEF/TCF-Komplexes ein zentraler Schritt in der Tumorentstehung.

In der Maus wurde gezeigt, das die Deletion des Gens für LEF-1 oder die Expression von β-Catenin-Mutanten, die gegenüber dem Abbau in der Zelle stabilisiert sind, unter anderem zur Störung der Entwicklung von Haarfollikeln führt. Interessanter weise führt die Expression von stabilisiertem β-Catenin zur Vermehrung der Anzahl der Haarfollikel (Gat U. et al., 1998. De novo hair follicie morphogenesis and hair tumors in mice expressing a truncated beta-catenin in skin. Cell 95:605-14) und die Inaktivierung von LEF-1 zur Störung der Entwicklung von Haarfollikeln, Brustdrüsen und Zähnen (van Genderen et al., 1994. Development of several organs that require inductive epithelial-mesenchymal interactions is impaired m LEF-1-deficient mice.Genes Dev. 8:2691-703 ). Diese Befunde weisen darauf hin, daß der Komplex von LEF/TCF und β-Catenin an diesen Entwicklungsprozessen beteiligt ist.

Es wurde bereits versucht, die Schlüsselrolle von β-Catenin in der Tumorentstehung für die Entwicklung von Tumortherapeutika auszunutzen. In den USA wurden zwei Patentanmeldungen vorgenommen, die als WO-Schriften veröffentlicht wurden. In WO 98/41631 (John Hopkins Universität - B. Vogelstein) wird die Beeinflussung von Interaktionen von β-Catenin, TCF-4 und dem Tumorsuppressor-Protein APC mit dem Ziel der Verhinderung von Krebsentstehung beansprucht. Dabei wurde gezeigt, daß Produkte von mutierten APC-Genen, die in Kolorektal-Tumoren nachgewiesen wurden, die β-Catenin/TCF-4-Transkriptionsaktivierung nicht mehr regulieren können. Weiterhin weisen Kolorektal-Tumore mit intakten APC-Genen Aktivierungsmutationen von β-Catenin im N-Terminus auf, was die Funktion der wichtigen Phosphorylierungsorte beeinflußt. Daraus wird abgeleitet, daß die Regulierung von β-Catenin für den Tumorsuppressorwirkung von APC kritisch ist und daß diese Regulierung durch Mutationen in APC oder in β-Catenin umgangen werden kann. Der Hauptanspruch betrifft das Intron-freie DNA-Molekül, welches für TCF-4 kodiert.

WO 98/42296 (Onyx Pharmaceuticals Inc. - Rubinfeld) betrifft Zusammensetzungen und Methoden zur Diagnose und zur Behandlung von Krankheiten, die durch β-Catenin/Transkriptionsfaktor-Interaktionen ausgelöst werden. Der Hauptanspruch der WO 98/42296 betrifft das isolierte, stabilisierte β-Catenin und seine Fragmente, solche Fragmente sind allerdings nicht angegeben. Die Möglichkeit, hydrophobe Taschen in der Nähe von essentiellen Bindungsstellen des β-Catenins aufzufinden, wird in der WO 98/42296 weder beschrieben noch vorgeschlagen.

In der DE 198 07 390.9 wurde des weiteren vorgeschlagen, Peptide oder davon abgeleitete Strukturen, die aus β- Catenin oder seinen Interaktionspartnern stammen und die Interaktionen spezifisch beeinflussen, zu finden.

Es ist somit eine Aufgabe der vorliegenden Erfindung, ein Verfahren zur Auffindung von Substanzen als neue Mittel zur Behandlung von Karzinomen bzw. aberranter Gewebs- und Organ entwicklung auf der Basis von Substanzen, die die Bindung von β-Catenin an LEF-1/TCF-Transkriptionsfaktoren, APC oder Conductin/Axin verhindern zur Verfügung zu stellen. Dabei soll die Interaktion von β-Catenin mit LEF/TCF-Transkriptionsfaktoren, APC und Conductin/Axin als Voraussetzung der Translokation und der Aktivität des Komplexes im Zellkern beeinflußt werden. Diese Aktivität muß zudem spezifisch sein, d.h. sie darf nicht gleichzeitig mit anderen Interaktionen von β-Catenin interferieren.

Die Erfindung wird gemäß den Ansprüchen realisiert, wesentliche Basis der Erfindung ist das überraschende Auffinden von separaten essentiellen Bindungsstellen dieser Interaktionspartner im β-Catenin-Molekül. Der Kemgedanke besteht darin, (1.) basierend auf den Kristallstrukturdaten und Oberflächenberechnungen für β-Catenin nach hydrophoben Taschen in der Nähe der essentiellen Bindungsstellen von LEF-1/TCF, APC oder Conductin/Axin zu suchen. Eine solche hydrophobe Tasche benachbart zur LEF/TCF Bindungsstelle wurde gefunden und charakterisiert (2.) Die hydrophoben Taschen benachbart der essentiellen Bindungsstellen werden zur Computer-gestützten Findung von niedermolekularen Substanzen genutzt, die in diesen Taschen binden können. (3.) Diese Substanzen werden im ELISA mit rekombinantem β-Catenin und einzelnen Interaktionspartnern auf Inhibitionseffektivität getestet. (4.) Auf den Kristall-Strukturdaten von β-Catenin basierend sollen die Substanzen durch Einführung von Seitengruppen optimiert werden, die zusätzlich durch Wechselwirkung mit den benachbarten Aminosäure-Resten der essentiellen Bindungsstellen (z.B. der LEF/TCF-Bindungsstelle: Lys 435, Arg 469, His 470, Lys 508 und Arg 515; oder der APC-Bindungsstelle für die 20 Aminosäure-Repeat-Domäne: Lys 345, Trp 383 oder der 15 Aminosäure-Repeat-Domäne Arg 386; oder der Conductin-Bindungsstelle: Phe 253, His 260, Lys 292) in ihrer Bindung stabilisiert werden.

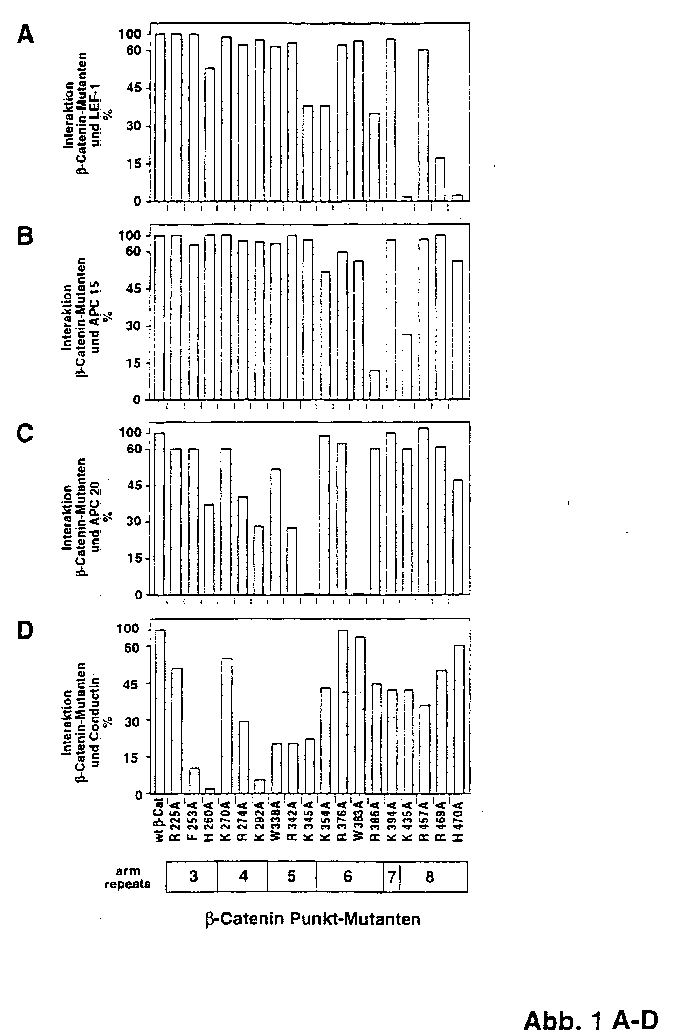

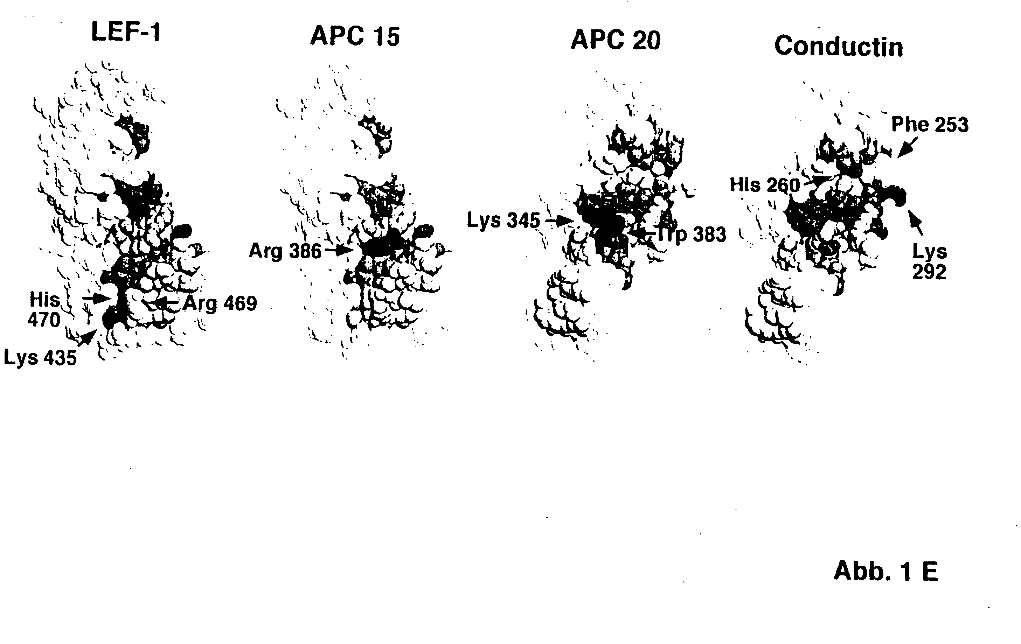

Im folgenden wird die Erfindung näher erläutert
In der ersten Realisierung der Erfindung wurden β-Catenin-Mutanten identifiziert, die die jeweilig essentielle Bindungsstelle für LEF-1/TCF-4, für die 20 Aminosäure-Repeats von APC oder für Conductin markieren (Abb. 1 ). Die Substitution einzelner basischer Aminosäure-Reste durch Alanin-Reste erzeugte diese Punkt-Mutanten. Da die essentiellen Bindungsstellen der Faktoren in getrennten Subregionen von β-Catenin lokalisieren, werden Substanzen die in der Nachbarschaft dieser Bindungsstellen binden nur spezifisch jeweils eine Interaktion beeinflussen. Diese Substanzen können gemäß der Erfindung für die Tumortherapie oder Geweberegeneration eingesetzt werden.

In einem zweiten Schritt wurden basierend auf den Röntgenkristall-Strukturdaten der Armadillo-Domäne von β-Catenin die Oberflächen in dieser Region berechnet. Durch Nutzung verschiedener Computerprogramme wie z.B. Grasp, SPOCK oder LUDI (MSI) wurde eine hydrophobe Tasche in der Nähe der Bindungsstelle für LEF-1/TCF-4 identifiziert (umrandet von den Aminosäuren Val 358, Met 363, Ala 391, Ala 392, Thr 393, Lys 394, Gln 395, Met 398, Leu 401, Leu 402, Ile 423, Asn 426, Leu 427, Thr 428, Cys 429, Asn 430, Asn 431, Asn 434, Met 437, Val 438). Diese hydrophobe Tasche bildet ein ideales molekulares Ziel für die Generation von Substanzen , die in dieser Tasche binden und Kontakte zur dicht benachbarten essentiellen Bindungsstelle herstellen. Aus energetischen Gründen ist diese Bindung favorisiert, da keine Hydrathülle zur Bindung verdrängt werden muß. Es ist möglich, daß ein Phenylalanin-Rest von LEF/TCF (Phe 24 von LEF-1 oder Phe 21 von TCF-4), der normalerweise für die Bindung an β-Catenin essentiell ist (DE 199 09 251 vom 22.2.99), in dieser Tasche bindet. Somit könnten die Substanzen einzig durch die Bindung in der Tasche diesen Kontaktpunkt in β-Catenin blockieren.
In einem dritten Schritt wurden computergestützt niedermolekulare Substanzen aus Molekül-Datenbanken in diese Tasche eingepaßt und aufgrund der Anzahl der stabilisierenden Wechselwirkungen mit β-Catenin ausgewählt. Diese Substanzen werden unten genannt (Tabelle: "Drug-Liste"). Die Grundstrukturen dieser niedermolekularen Verbindungen sollen nach der experimentellen Überprüfung ihrer Inhibitions-Funktion (z.B. im ELISA, Tabelle: "Positiv-Liste") modifiziert werden, um ihre Funktion zu optimieren. Hierfür ist die Idee leitend, das die Modifikation der Substanzen z.B. durch Einführung saurer Gruppen, zusätzliche Wechselwirkungen mit den basischen Resten der Aminosäuren Lys 435, Arg 469, His 470, Lys 508 oder Arg 515 von β-Catenin ermöglicht.

Da diese Reste die essentielle Bindungsstelle der LEF/TCF-Faktoren markieren, sollten diese zusätzlichen Wechselwirkungen die Effektivität der Substanzen verstärken.
Die Anwendung dieser Strategie auf alle essentiellen Bindungsstellen von β-Catenin ermöglicht die Generierung neuer Pharmaka, die durch spezifische Beeinflußung der jeweiligen Interaktion von β-Catenin z.B. mit den Transkriptionsfaktoren LEF/TCF, dem Tumorsuppressor-Genprodukt APC oder mit Conductin die Entwicklung von Tumoren inhibieren oder zur Regeneration der Haut und zur Förderung der Haarbildung eingesetzt werden. Diese Möglichkeiten der Beeinflußung ergeben sich aus den publizierten Befunden zur Funktion der jeweiligen Interaktion von β-Catenin mit seinen Bindungspartnern in diesen Entwicklungsprozessen.

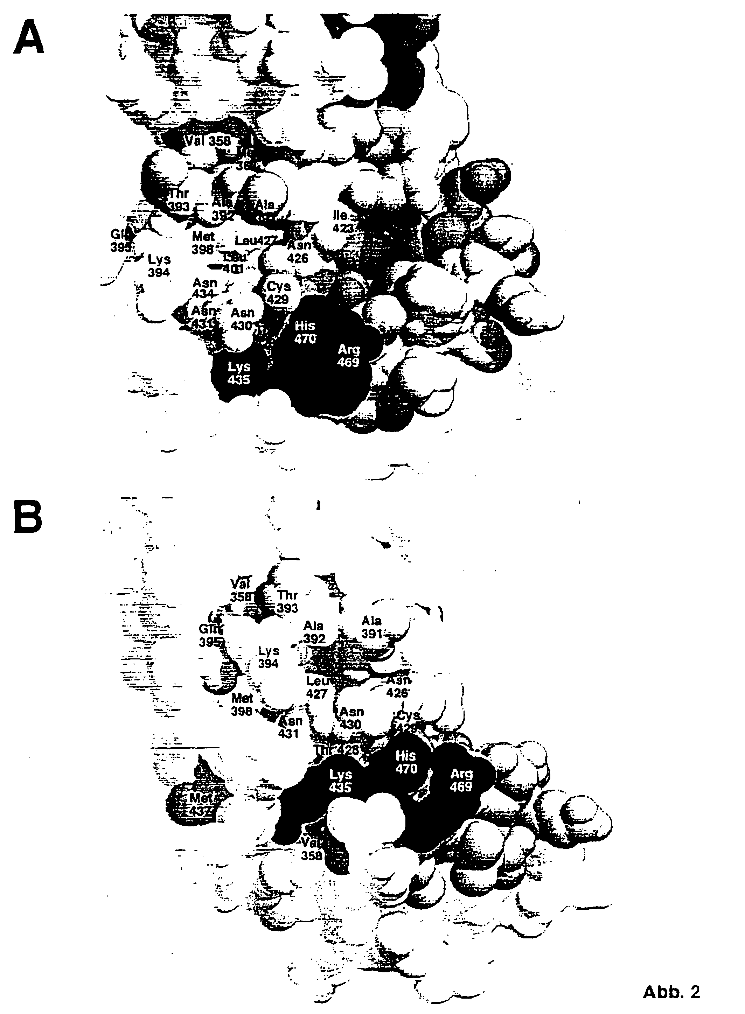

Im Einzelnen wurden folgende Untersuchungen durchgerührt:
1. Identifizierung von separaten, essentiellen Bindungsstellen von β-Catenin für die Interaktion mit LEF-1/TCF-4, mit den 20 und 15 Aminosäure-Repeats von APC oder Conductin/Axin
   Basierend auf der Röntgen-Kristall-Strukturanalyse der Armadillo-Domäne von β-Catenin (Huber et al., 1997) wurden ausgewählte basische und aromatische Aminosäure-Reste durch Alanin-Reste substituiert und die Punktmutanten auf Interaktion mit den Bindungspartnern von β-Catenin im Hefe-2-Hybrid-System analysiert (Abb. 1). Die Mutationen, welche spezifische Interaktionen blockieren, bilden Cluster in separaten Subregionen der Domäne. Sie markieren die essentiellen Kontaktpunkte in den spezifischen Bindungsstellen für die Interaktion mit LEF/TCF (Lys 435, Arg 469, His 470, Lys 508, Arg 515), mit den 20 Aminosäure-Repeats von APC (Trp 383, Lys 345), mit den 15 Aminosäure-Repeats von APC (Arg 386), oder mit Conductin (Phe 253, His 260, Lys 292). Die erweiterten Interaktionsstellen sind der Abb 1 zu entnehmen.
2. Analyse der Moleküloberfläche von β-Catenin im Bereich der essentiellen Bindungsstelle für LEF/TCF
   Ausgehend von den Daten zur Röntgenkristallstruktur von β-Catenin wurde mit den Programmen Grasp und LUDI (MSI) die Moleküloberfläche im Bereich der Bindungsstelle berechnet. Es ist hierdurch gelungen eine hydrophobe Tasche zu identifizieren (Abb.2), die von den folgenden Aminosäuren umrandet ist: Val 358, Met 363, Ala 391, Ala 392, Thr 393, Lys 394, Gln 395, Met 398, Leu 401, Leu 402, Ile 423, Asn 426, Leu 427, Thr 428, Cys 429, Asn 430, Asn 431, Asn 434, Met 437, Val 438. Diese Tasche lokalisiert in engster Nachbarschaft zu der essentiellen Bindungsstelle für LEF/TCF. Sie ist von großer Bedeutung für die Computer-gestützte Durchmusterung von Substanzbibliotheken zur Selektion von Molekülen, die in dieser Tasche binden. Die Idee primär hydrophobe Wechselwirkungen in der Tasche zum Ausgangspunkt zu wählen, hat den energetischen Vorteil, das die Substanzen nicht mit der Hydrathülle der geladenen Aminosäure-Reste der Oberfläche kompetieren müssen. Diese Substanzen stellen somit nach gezielter Modifikation potente Therapeutika zur Behandlung von Tumoren dar, da die Interaktion von LEF/TCF mit β-Catenin ein frühes Ereignis zur Entstehung von Tumoren darstellt. Sie wurden experimentell auf ihre Fähigkeit zur Hemmung der onkogenen Interaktion getestet (ELISA) und sollen modifiziert werden. Das molekulare Modellieren der Substanzen in der Tasche basiert auf der Idee, die Wechselwirkungen in der Tasche selbst zu stabilisieren und zusätzliche Kontakte zu den essentiellen Aminosäure-Resten der LEF/TCF-Bindungsstelle zu schaffen. Durch diese Strategie sollen die Substanzen in ihrer Wirkung optimiert werden.
3. Identifizierung von Substanzen die in der hydrophoben Tasche binden
   Es wurden Computer-gestützt Substanzbibliotheken durchmustert und deren Moleküle auf stabilisierende Wechselwirkungen in der hydrophoben Tasche ausgewählt. Die ausgewählten Substanzen wurden auf Hemmung der Komplexbildung von β-Catenin und LEF-1 im ELISA experimentell getestet. Die Grundstrukturen der ausgewählten und im ELISA wirksamen Substanzen bilden unterschiedliche Molekülklassen, die im folgenden dargestellt sind:
   Molekülklasse I:
      A: Cephalosporine von gleicher Grundstruktur wie die Substanz "Cefamandole" mit einer für die Hemmung der Komplexbildung von β-Catenin und LEF/TCF (IC₅₀= 25-100µM) essentiellen Kernstruktur. Diese Kernstruktur besteht aus einem aromatischem Ring (beim Cefamandole am Kohlenstoffatom 17, Numerierung siehe "Positiv-Liste")), einer organischen Komponente, die der Struktur des Ala-Ala Dipeptides ähnelt, und einem β-Lactam- und Thiazolring. Mit dem aromatischen Ring wurde die Substanz in der beschriebenen hydrophoben Tasche eingepaßt. Verwandte Cephalosporine ohne diesen aromatischen Ring weisen keine Hemmwirkung auf. Zusätzlich wurden stabilisierende Wasserstoffbrücken zwischen einer Carbonylgruppe (Kohlenstoffatom 17) der Substanz mit dem Ser 389 von β-Catenin und einer Carboxylgruppe (Kohlenstoffatom 8, am Thiazolring) und dem Lys 394 von β-Catenin berechnet. Weitere Vertreter dieser Molekülklasse mit experimentell bestätigter Hemmung der Komplexbildung sind die Cephalosporine Cefamandole-Nafate, Cefsulodine und Cefadroxil (IC₅₀= 50-100µM). Grundsätzlich wird der Anspruch auf alle Cephalosporine mit dergleichen Kemstruktur wie der des Cefamandoles als Leitstruktur zur Entwicklung eines Heilmittels oder unmodifiziert als Heilmittel zur Bekämpfung von Tumoren und Krebs erhoben.
      B: Die Substanz AC-(6-O-Stearoyl)-Muramyl-Ala-D-Isoglutamine gehört der Struktur nach zu einer anderen Subklasse von Inhibitoren der Komplexbildung (IC₅₀= 100µM).
      C: Die Substanz 3,6-Dihydroxybenzonobomane gehört der Struktur nach zu einer anderen Subklasse von Inhibitoren der Komplexbildung (IC₅₀= 100µM).
   Molekülklasse II:
      Substanzen die ebenfalls in der hydrophoben Tasche benachbart zur essentiellen Bindungsstelle von β-Catenin für LEF/TCF binden, die jedoch nicht die Komplexbildung hemmen, da sie entweder zu klein sind um die Komplexbildung zu stören oder in anderer Ausrichtung als die Substanzen der Molekülklasse 1 in, der Tasche binden. Für diese Substanzen wurde jedoch im Experiment eine starke Beeinflussung der Wirkung der Moleküle der Klasse I durch Kompetition der Bindung in der Tasche belegt. Diese Moleküle dienen somit ebenfalls als Leitstrukturen zur Modifikation und Entwicklung potenter Inhibitoren der Komplexbildung von LEF/TCF in Tumoren.

Die Erfindung soll nachfolgend durch Ausführungsbeispiele näher erläutert werden.
1. Herstellung und Testen von Mutanten von β-Catenin, die die essentiellen Bindungsstellen für LEF-1, APC und Conductin markieren.
   Die Mutagenese von β-Catenin in den Armadillo-Repeats 3-8 wurde mit dem "Mutagenese Kit" der Firma Clontech nach dem Protokoll des Herstellers durchgeführt und die Mutanten durch Sequenzierung überprüft. In allen Mutanten wurde die ursprüngliche Aminosäure durch Alanin substituiert. Für die Analyse der Interaktionen wurde die für die Aminosäuren Leu218-Leu781 kodierende cDNA von humanen β-Catenin (Armadillo-Repeat 3 bis zum C-terminalen Ende des Proteins) oder seinen Mutanten in den Fusionsvektor für die Aktivierungsdomäne von Gal-4 (pGAD424, Clontech) kloniert. Die cDNA für die Bindungsdomänen der Interaktionspartner wurde in den LexA-Fusionsvektor BTM 116 kloniert. Hierfür wurde die cDNA von LEF-1 für die Aminosäuren 1-99, von Conductin für die Aminosäuren Ala342-Arg465, von humanen APC für die Aminosäuren His1012-Glu1215 (APC 15 Aminosäure-Repeats) und für die Aminosäuren Ser1259-Asp1400 (APC 20 Aminosäure-Repeats) mit entsprechenden Primem PCR amplifiziert. Die Interaktion der Lex-A-Hybride mit β-Catenin und seinen Mutanten wurde anhand der b-Galaktosidase- Reporteraktivität im Hefe 2-Hybrid System (Protokoll: "Matchmaker", Clontech) quantifiziert (Abb. 1).
2. Identifizierung einer hydrophoben Tasche in β-Catenin benachbart zur essentiellen Bindungsstelle für LEF-1. Zur Berechnung der Moleküloberfläche von β-Catenin wurden verschiedene die Computer-Programme wie z.B. GRASP, SPOCK und LUDI von MSI benutzt.
3. Identifizierung von Substanzen die in der hydrophoben Tasche bei der essentiellen Bindungsstelle für LEF-1 binden.
   Für die Suche von Substanzen die in der hydrophobe Tasche binden können wurden verschiedene Substanz-Bibliotheken zum Beispiel die "Available Chemicals Database, ACD" von MSI und die Programme LUDI, SPOCK und GRASP benutzt.
4. ELISA zum Test der Substanzen auf Hemmung der Komplexbildung von β-Catenin und LEF-1.

Für den Test der ausgewählten Substanzen auf Hemmung der LEF-1 β-Catenin Interaktion wurden zuerst beide Proteine in Bakterien rekombinant mit N-terminalen Histidin-Sequenzen hergestellt und durch Nickel-Chromatographie gereinigt (Behrens et al. 1996). Ca. 50 ng LEF-1 wurde an den Näpfen von ELISA-Platten für 60 Minuten bei Raumtemperatur in PBS/BSA (0.1 mg BSA/ml) adsorbiert. Anschließend wurden die Näpfe mit 2.5% Magermilch-Pulver/0.5% BSA in PBS für 2 Stunden bei 8° C abgedeckt. Alle weiteren Schritte erfolgten bei Raumtemperatur in PBS/BSA (10 µg BSA/ml). Nach dem Waschen der Näpfe mit PBS wurden die in DMSO gelösten Substanzen ad 10% DMSO final und den angegebenen Endkonzentrationen der Substanzen zugegeben. Die Inkubation mit 50-100 ng β-Catenin wurden für 15 Minuten in PBS/BSA (0.5 mg BSA/ml) durchgeführt. Die Komplexbildung von LEF-1 und β-Catenin wurde durch den Antikörper PA2 gegen den Carboxy-Terminus von β-Catenin nachgewiesen (Hülsken et al. 1994). PA2 wurde für 15 Minuten in einer Titerverdünnung von 1:10000 in 1% BSA/PBS zugegeben. Nach dem Waschen der Näpfe mit PBS erfolgte die Quantifizierung der Komplexbildung mit Peroxidase konjugierte Nachweisantikörper (1:2000 in 1% BSA/PBS, Dianova) und durch photometrische Messung des Umsatzes von o-Phenylendiamin als Substrat bei 450 nm (Ultramark ELISA-Reader, BioRad). Die Substanzen wurden in Konzentrationen von 1 µM bis 10 mM eingesetzt. Als Kontrolle der spezifischen Inhibition der Komplexbildung von LEF-1 und β-Catenin, wurde β-Catenin in den Näpfen adsorbiert und nach Inkubation mit den Substanzen nachgewiesen. Substanzen, die in der hydrophoben Tasche von β-catenin binden ohne die Komplexbildung mit LEF-1 zu behindern, wurden aufgrund ihrer Konkurrenz um diese Bindungsstelle mit den Substanzen mit Hemmwirkung identifiziert. Hierfür wurde z.B. Cefamandole als Substanz mit Hemmwirkung in seiner halbmaximal wirksamen Konzentration (IC₅₀= 25 µM) mit jeweils einer der anderen Substanzen in dergleichen Konzentration (100 µM) in einem Bindungsansatz im ELISA zusammen eingesetzt. Substanzen, die am gleichen Ort wie Cefamandole binden, aber allein eingesetzt die Komplexbildung nicht hemmen, können die Bindung von Cefamandole beeinflussen.

### Legende zu den Abbildungen und Tabellen

Abb.1 Identifizierung der essentiellen Bindungsstellen von β-Catenin für LEF-1, die 20 Aminosäure-Repeats von APC oder Conductin
   (A-D) Interaktion von β-Catenin-Mutanten mit LEF-1, APC (15- und 20 Aminosäure-Repeats) und Conductin im Hefe-2-Hybrid-System. Die durch Alanin substituierten Aminosäure-Reste in den β-Catenin-Punktmutanten und deren Position in den Arm-Repeats 3-8 sind unten angegeben. Die Interaktion wurde durch Bestimmung der β-Galaktosidase-Reporteraktivität quantifiziert und ist im Verhältnis zur Interaktion mit Wildtyp β-Catenin angegeben. (E) Darstellung der separaten essentiellen Bindungsstellen in der Armadillo-Domäne von β-Catenin (RasMol).
Abb.2 Charakterisierung einer hydrophoben Tasche benachbart zur essentiellen Bindungsstellen von β-Catenin für LEF-1/TCF
   (A) Aufsicht der hydrophoben Tasche in der Moleküloberfläche von β-Catenin (RasMol). Die Tasche wird durch die in orange oder gelb markierten Aminosäuren umrandet. Die Aminosäurereste der essentiellen Bindungsstelle für LEF/TCF sind blau markiert. Die entsprechenden Aminosäuren wurden gekennzeichnet. (B) Seitenansicht der hydrophoben Tasche.

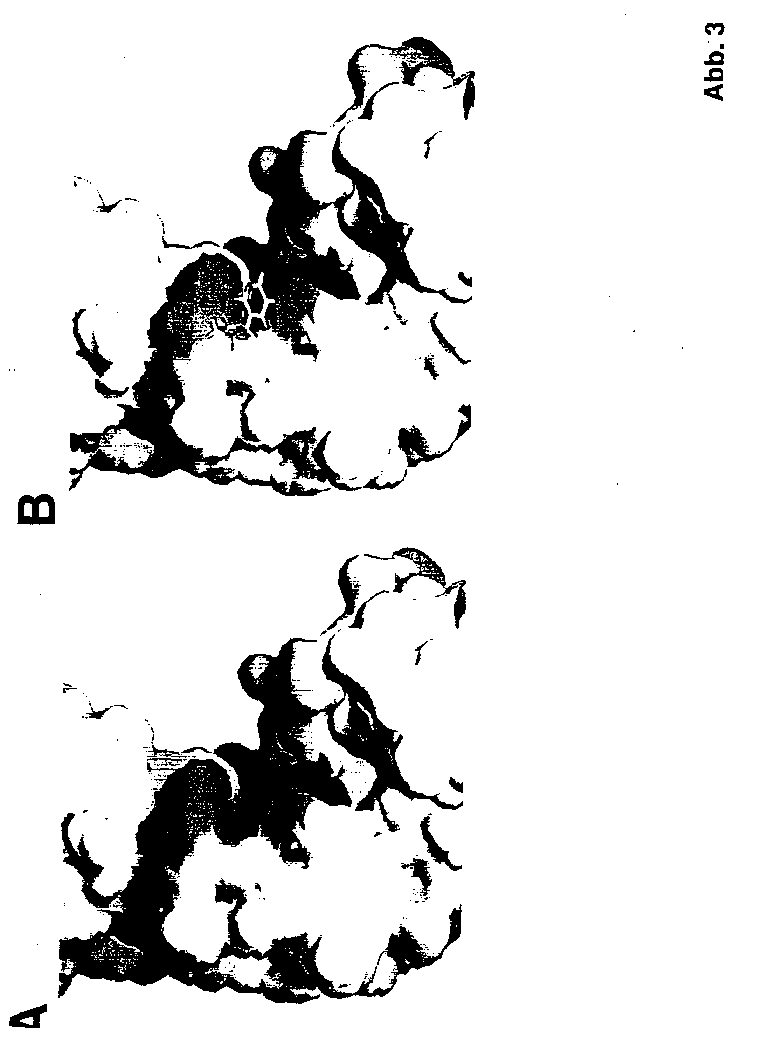

Abb.3 Substanzen, die in der hydrophoben Tasche von β-Catenin binden
   (A) Oberflächendarstellung der Region der hydrophoben . Tasche (Grasp). Die Aminosäurereste der essentiellen Bindungsstelle für LEF/TCF sind blau markiert (für die Mutationen, die die Interaktion von β-Catenin mit LEF/TCF blockieren: Lys435, Arg 469 und His 470). (B) Im β-Catenin-Molekül ist eine der niedermolekularen Substanzen dargestellt, die in der Tasche bindet.

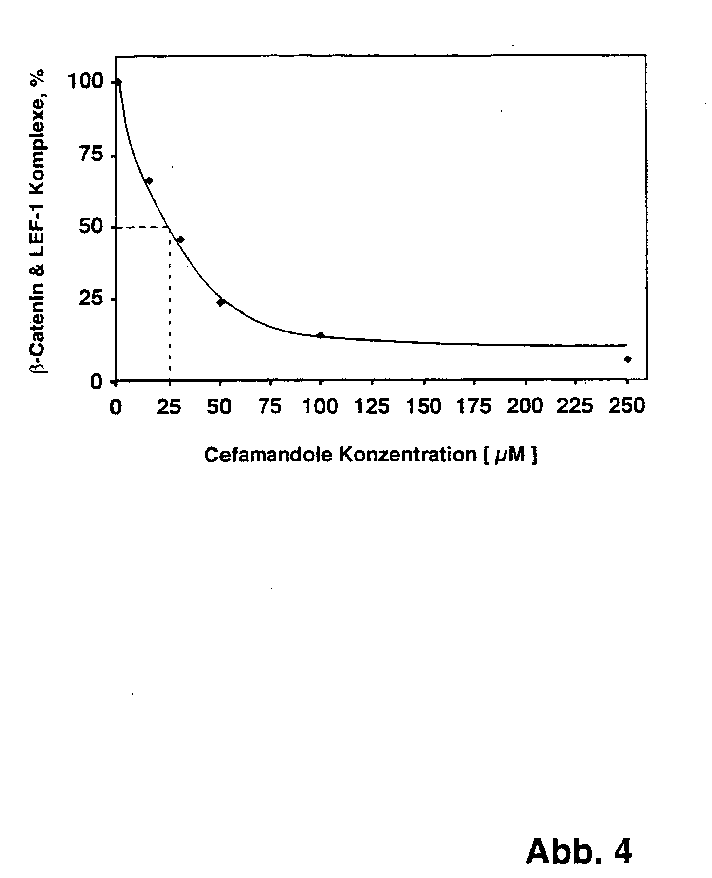

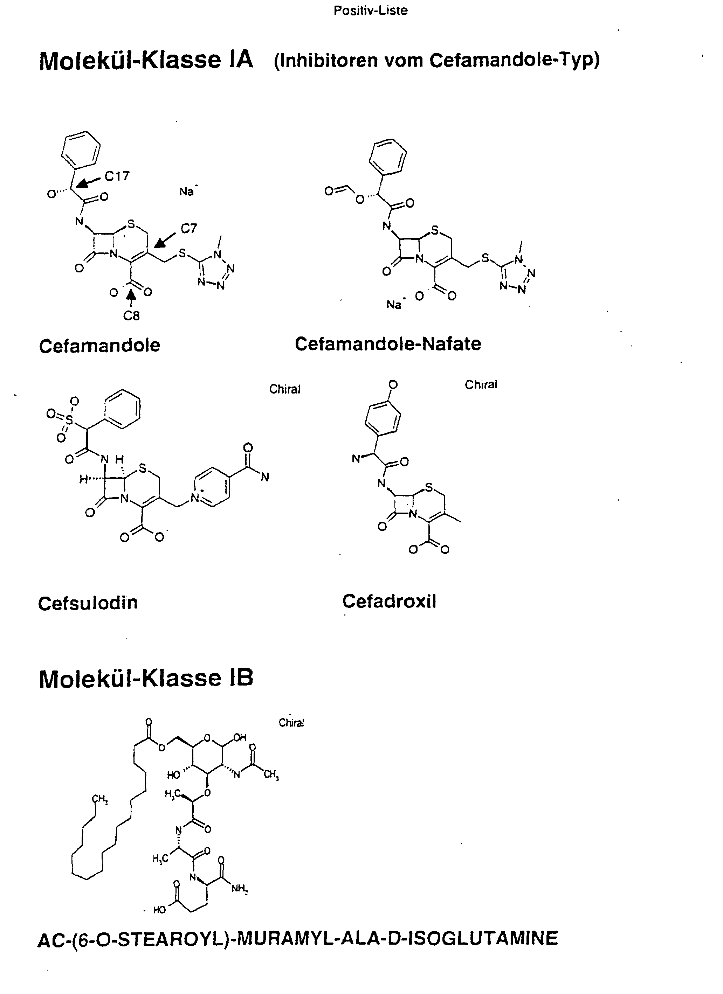

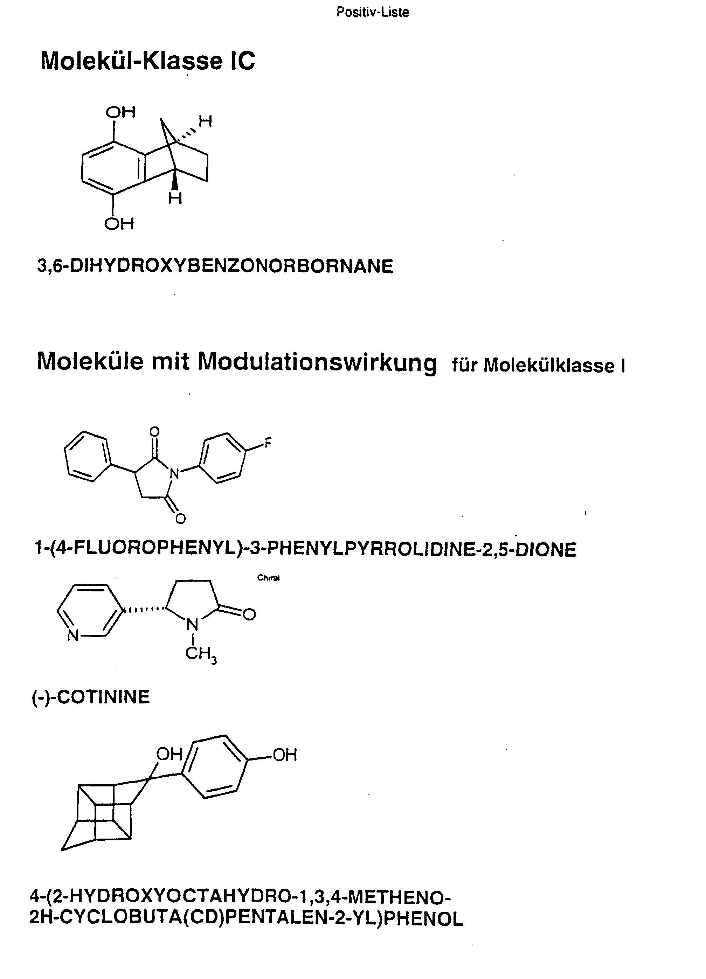

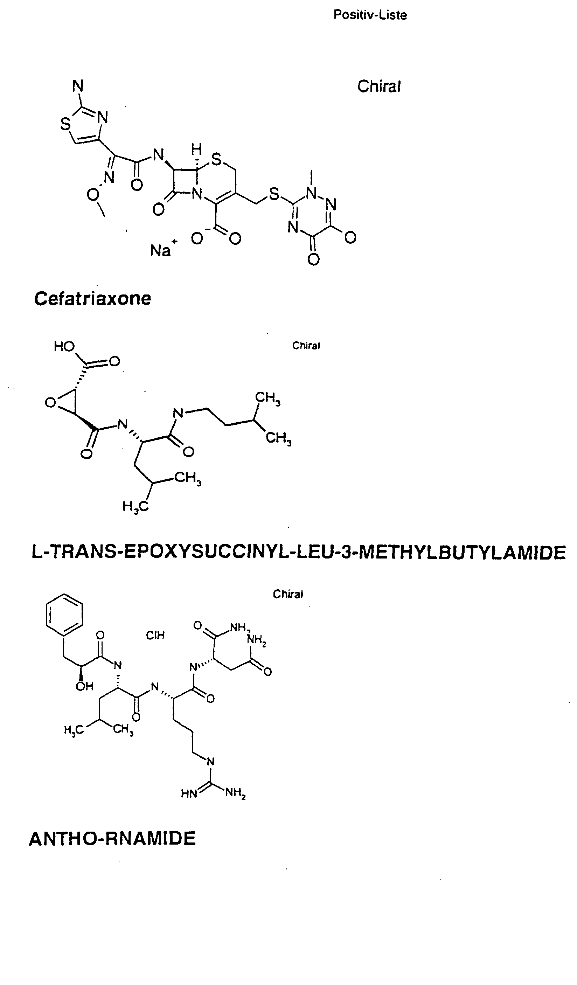

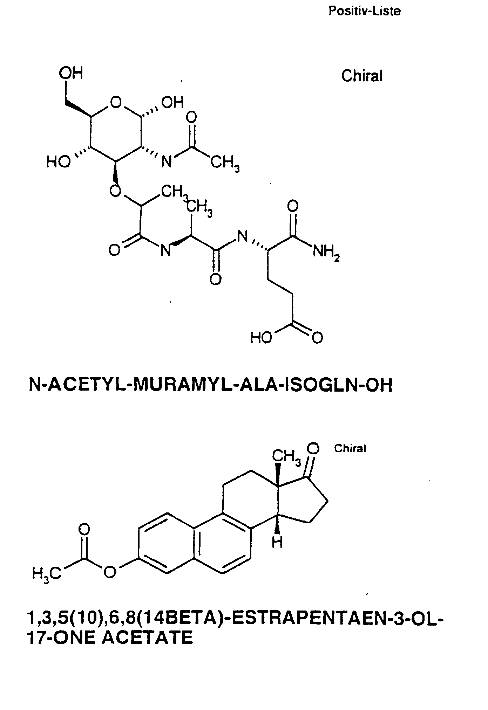

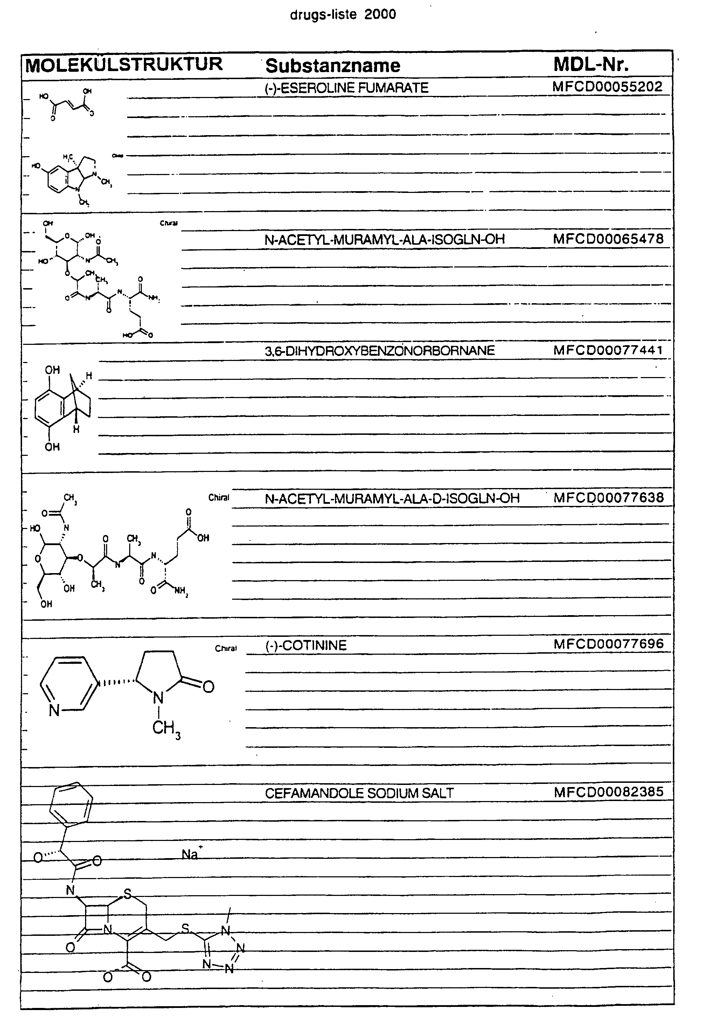

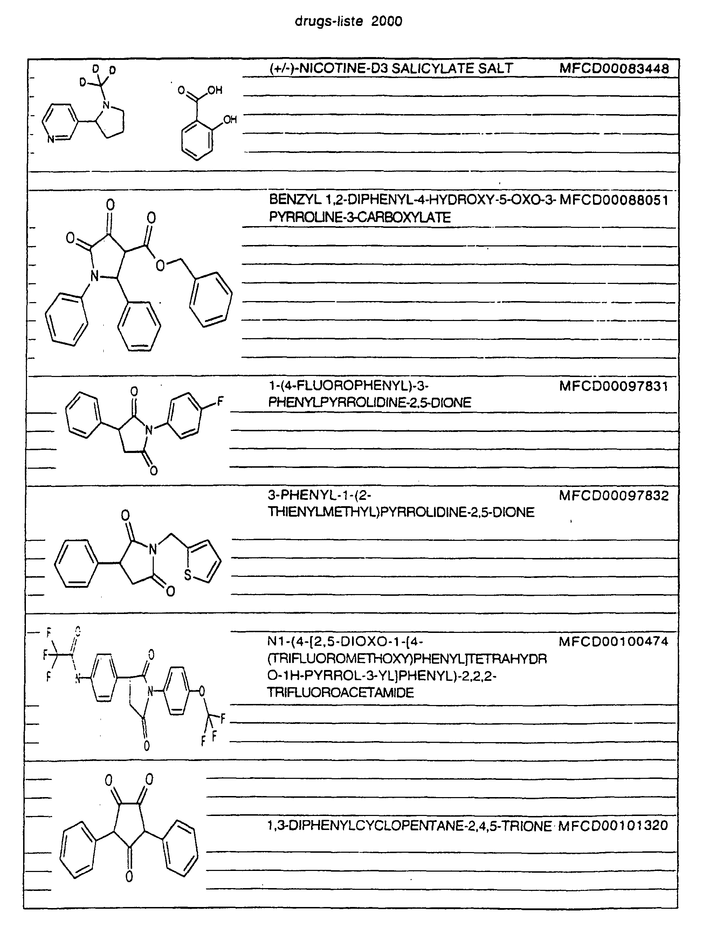

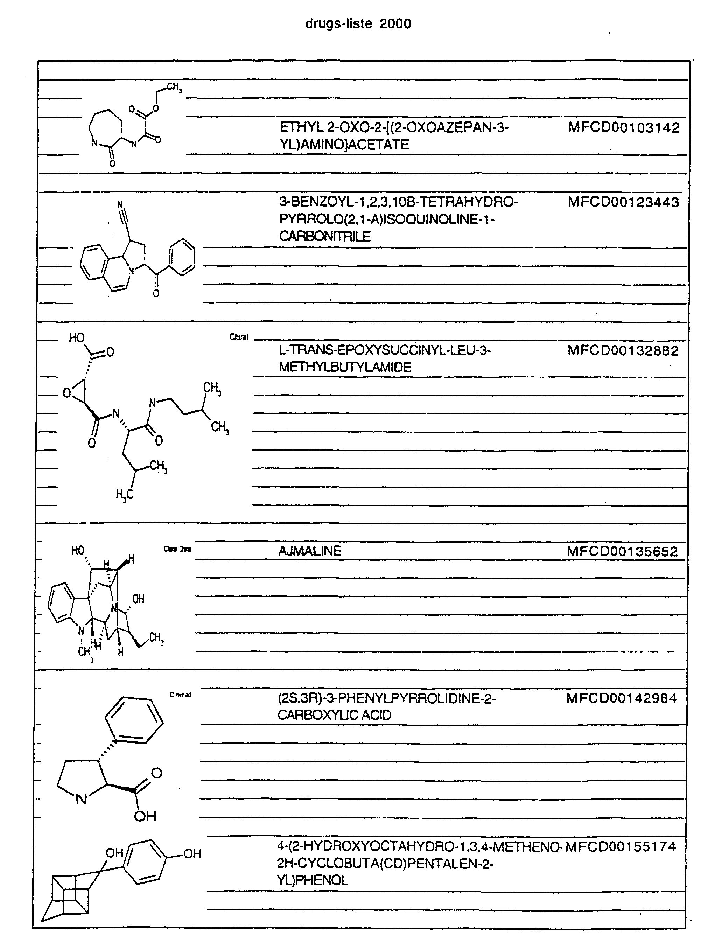

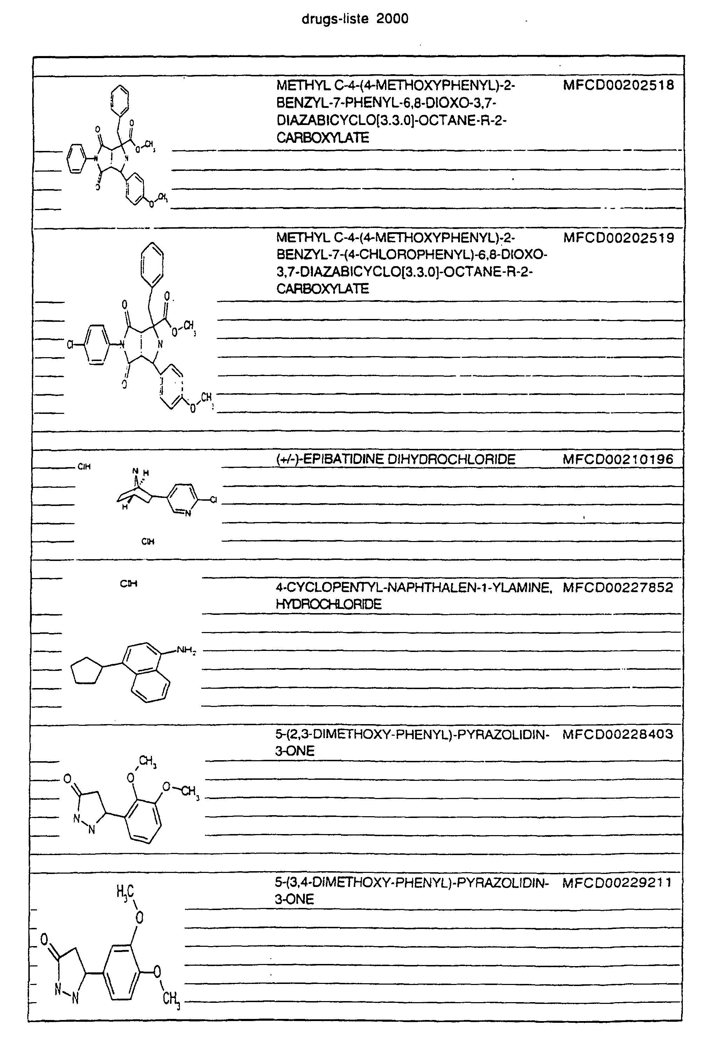

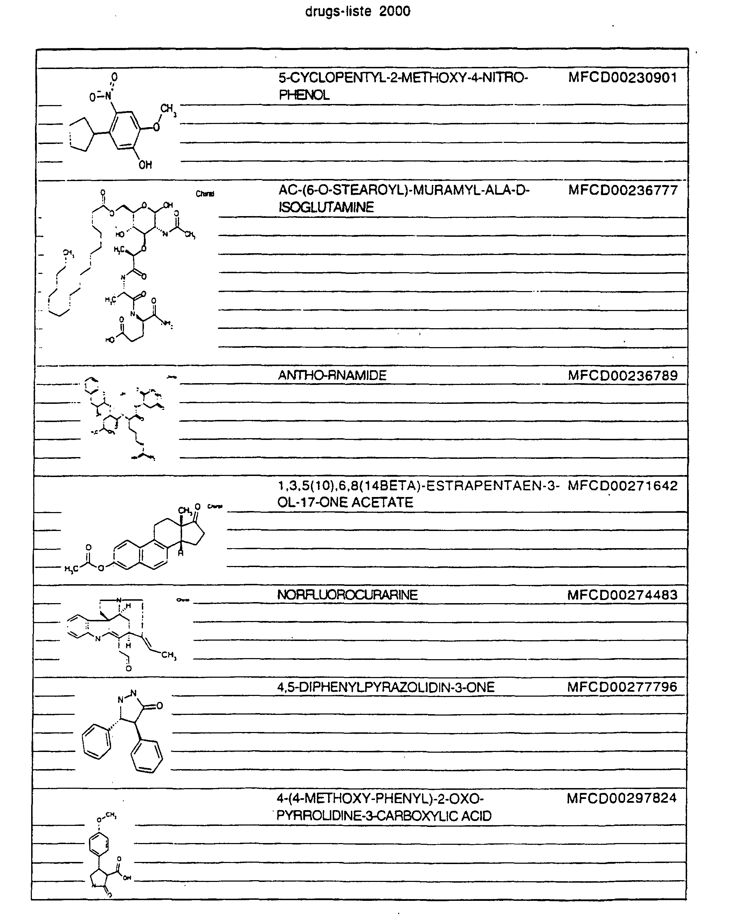

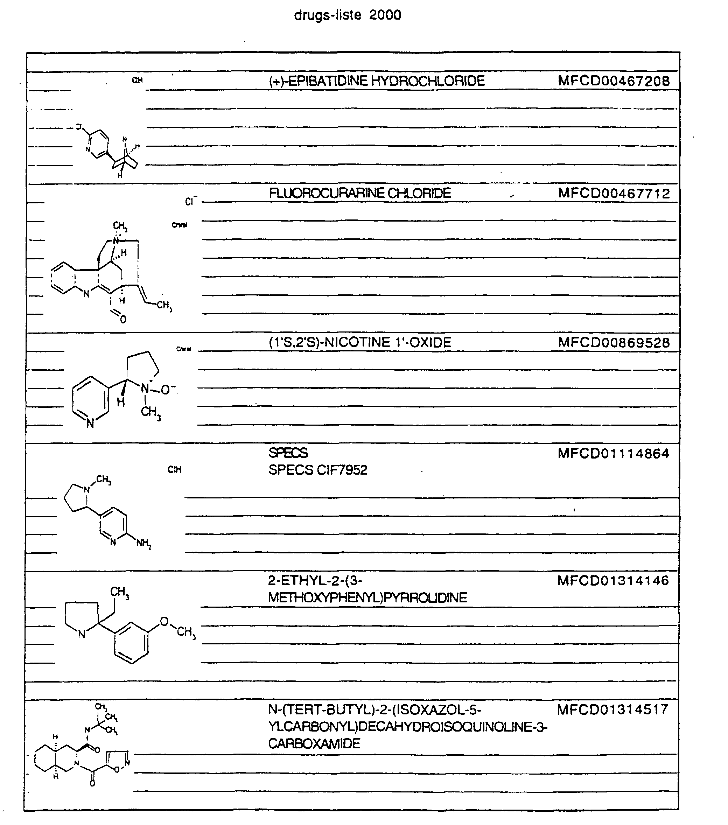

Abb. 4 Cefamandole als Vertreter der Molekülklasse I hemmt die Komplexbildung von LEF-1 und β-Catenin im ELISA.
   Ansteigende Konzentrationen von Cefamandole (15-250 µM) führen zur Abnahme der Komplexbildung von rekombinant hergestelltem und gereinigtem LEF-1 und β-Catenin Protein im ELISA (IC50=25 µM).

Tabelle 1: Substanzen die nach Computer-Berechnungen (u.a. LUDI/MSI) potentiell in der hydrophoben Tasche binden ("Drugs-Liste")

Tabelle 2: "Positivliste" von Substanzen die die Komplexbildung von b-Catenin und LEF-1 im ELISA hemmen oder die Wirkung von Cefamandole beeinflussen.

## Patentansprüche

1. Verfahren zum Auffinden von Substanzen, die die Bindung von β-Catenin selektiv an LEF-1/TCF-Transkriptionsfaktoren, APC oder Conductin/Axin verhindern, wobei
a) im β-Catenin-Molekül in Nachbarschalt zu essentiellen Bindungstellen hydrophobe Taschen identifiziert werden, und
b) nachfolgend die in diese Tasche passenden therapeutischen Substanzen synthetisiert und getestet werden.

2. Verfahren nach Anspruch 1, wobei
a) β-Catenin-Mutanten identifiziert werden, die die jeweilige essentielle Bindungsstelle für LEF-1/TCF-Transkriptionsfaktoren, APC oder Conductin markieren,
b) die Oberflächen in dieser Region basierend auf den Röntgenkristall-Strukturdaten der Armadillo-Domäne berechnet und
c) dadurch die vorhandenen hydrophoben Taschen identifiziert werden,
d) niedermolekulare Verbindungen in diese Tasche eingepaßt und aufgrund der stabilisierenden Wechselwirkungen mit β-Catenin und selektiven Hemmung oder Förderung der Komplexbildung mit LEF/TCF, APC oder Conductin im biologischen Test ausgewählt werden, und
e) diese Verbindungen, gegebenenfalls durch Einführung saurer Gruppen, weiter modifiziert werden.

3. Verfahren nach Anspruch 1 oder 2, wobei
a) die β-Catenin-Mutanten Lys 435, Arg 469, His 470, Lys 508, Arg 515 die die essentielle Bindungsstelle für LEF-1 markieren,
b) die β-Catenin-Mutanten Phe 253, His 260, Lys 292, die die essentielle Bindungsstelle für Conductin markieren,
c) die β-Catenin-Mutanten Lys 345, Trp 383, Arg 386, die die essentielle Bindungsstelle für APC markieren, identifiziert werden,
d) die Moleküloberfläche mit dem Programm Grasp, Spock, LUDI (MSI) oder andere zur Berechnung von Moleküloberflächen geeignete berechnet wird,
e) eine hydrophobe Tasche, umrandet von den Aminosäuren Val 358, Met 363, Ala 391, Ala 392, Thr 393, Lys 394, Gln 395, Met 398, Leu 401, Leu 402, Ile 423, Asn 426, Leu 427, Thr 428, Cys 429, Asn 430, Asn 431, Asn 434, Met 437, Val 438, benachbart zur LEF-1/TCF-Bindungsstelle identifiziert wird, und
f) Verbindungen, die ausgewählt sind aus der Gruppe bestehend aus Cefamandol, Cefamandol-Nafat, Cefamandol Natriumsalz, Cefsulodin, Cefadroxil, Cefatriaxon, AC-(6-O-Stearoyl)-muramyl-Ala-D-isoglutamin, 3,6-Dihydroxybenzonoboman, 1-(4-Fluorphenyl)-3-phenylpyrrolidin-2,5-dion, (-)-Cotinin, 4-(2-Hydroxyoctahydro-1,3,4-metheno-2H-cyclobuta(CD)pentalen-2-yl)phenol, Antho-RNAmid, L-transepoxysuccinyl-Leu-3-methylbutylamid, N-Acetyl-muramyl-Ala-isoGln-OH, 1,3,5(10),6,8(14beta)-Estrapentaen-3-ol-17-on-acetat, (-)-Eserolinfumarat, N-Acetylmuramyl-AlaD-isoGln-OH, Ethyl-2-oxo-2-[(oxoazepan-3-yl)amino]azetat, 3-Benzoyl-1,2,3,10-B-tetrahydropyrrolo(2,1-A)isochinolin-1-carbonitril, Ajmalin, (2S,3R)-3-Phenylpyrrolidin-2-carboxylsäure, 4-(2-Hydroxyoctahydro-1,3,4-metheno-2Hcyclobuta(CD)pentalen-2-yl)phenol, Methyl-C-4-(4-methoxyphenyl)-2-benzyl-7-phenyl-6,8-dioxo-3,7-diazabicyclo[3.3.0]-octan-R-2-carboxylat, Methyl-C-4-(4-methoxyphenyl)-2-benzyl-7-(4-chlorphenyl)-6,8-dioxo-3,7-diazabicyclo[3.3.0]-octan-R-2-carboxylat, (+/-)-Epibatidindihydrochlorid, (+)-Epibatidinhydrochlorid, 4-Cyclopentyl-naphthalin-1-ylaminhydrochlorid, 5-(2,3-Dimethoxy-phenyl)-pyrazolidin-3-on, 5-(3,4-Dimethoxy-phenyl)-pyrazolidin-3-on, 5-Cyclopentyl-2-methoxy-4-nitrophenol, 1,3,5(10),6,8(beta)-Estrapentaen-3-ol-17-onacetat, Norfluorcurarin, Fluorcurarinchlorid, 4,5-Diphenylpyrazolidin-3-on, 4-(4-Methoxy-phenyl)-2-oxo-pyrrolidin-3-carboxylsäure, 2-Ethyl-2-(3-Methoxyphenyl)pyrrolidin, N-(tert-Butyl)-2-(isoxazol-5-ylcarbonyl)decahydroisochinolin-3-carboxamid, (+/-)-Nicotin-D3-salicylat, Benzyl-1,2-diphenyl-4-hydroxy-5-oxo-3-pyrrolin-3-carboxylat, (1'S,2'S)-Nicotin-1'-oxid, Specs CIF7952, 3-Phenyl-(2-thienylmethyl)pyrrolidin-2,5-dion, N1-(4-[2,5-Dioxo-1-[4-(trifluormethoxy)phenyl]tetrahydro-1-H-pyrrol-3-yl]phenyl)-2,2,2-trifluoracetamid und 1,3-Diphenylcyclopentan-2,4,5-trion, in die identifizierte hydrophobe Tasche eingepaßt werden und anschließend mit ELISA auf ihre Inhibierung der Komplexbildung von β-Catenin und seinen Bindungspartnern oder die Beeinflussung der Bindung von Cefamandol an β-Catenin experimentell überprüft und anschließend durch chemische Modifikation optimiert werden.

4. Verfahren nach einem der Ansprüche 1 bis 3, weiterhin umfassend Mischen der in eine Tasche passenden therapeutischen Substanz mit einem geeigneten pharmazeutischen Träger.

5. Verwendung eines Verfahrens nach einem der Ansprüche 1 bis 4 im Rahmen der Herstellung einer pharmazeutischen Zusammensetzung zur Behandlung von menschlichen Erkrankungen, Tumoren, wie Kolonkarzinomen und Melanomen, zur Gewebsregeneration oder zur Förderung des Haarwuchses.

## Claims

1. Method for identifying substances that selectively inhibit the binding of β-catenin to LEF-1/TCF-transcription factors, APC or conductin/axin, wherein
a) hydrophobic pockets in the neighbourhood of essential binding positions are identified in the β-catenin molecule, and
b) subsequently, therapeutic substances fitting into these pockets are synthesised and tested.

2. Method according to claim 1, wherein
a) β-catenin mutants are identified that mark the essential binding position for LEF-1/TCF transcription factors, APC or conductin, respectively,
b) the surfaces in this region are calculated based on the X-ray crystal structural data of the Armadillo domain, and
c) thereby the hydrophobic pockets as present are identified,
d) low molecular weight compounds are fitted into these pockets and selected in the biological test, based on the stabilising interactions with β-catenin and selective inhibition or promotion of the complex formation with LEF/TCF, APC or conductin, and
e) these compounds are further modified, optionally by introducing acidic groups.

3. Method according to claim 1 or 2, wherein
a) the β-catenin mutants Lys 435, Arg 469, His 470, Lys 508, Arg 515 that mark the essential binding position for LEF-1,
b) the β-catenin mutants Phe 253, His 260, Lys 292 that mark the essential binding position for conductin,
c) the β-catenin mutants Lys 345, Trp 383, Arg 386 that mark the essential binding position for APC are identified,
d) the surface of the molecule is calculated using the program Grasp, Spock, LUDI (MSI) or other programs that are suitable for calculating surfaces of molecules,
e) a hydrophobic pocket that is surrounded by the amino acids Val 358, Met 363, Ala 391, Ala 392, Thr 393, Lys 394, Gln 395, Met 398, Leu 401, Leu 402, Ile 423, Asn 426, Leu 427, Thr 428, Cys 429, Asn 430, Asn 431, Asn 434, Met 437, Val 438 in the neighbourhood to the LEF-1/TCF binding position is identified, and
f) compounds that are selected from the group consisting of cefamandol, cefamandolnafat, cefamandol sodium salt, cefsulodin, cefadroxil, cefatriaxon, AC-(6-O-stearoyl)-muramyl-ala-D-isoglutamic acid, 3,6-dihydroxybenzonobomane, 1-(4-fluorophenyl)-3-phenylpyrrolidine-2,5-dione, (-)-cotinine, 4-(2-hydroxyoctahydro-1,3,4-metheno-2H-cyclobuta(CD)pentalene-2-yl)phenol, antho-RNAmid, L-trans-epoxysuccinyl-leu-3-methylbutylamide, N-acetyt-muramyl-ala-isogln-OH, 1,3,5(10),6,8(14beta)-estrapentaene-3-ol-17-one-acetate, (-)-eserolinfumarate, N-acetyl-muramyl-alaD-isogln-OH, ethyl-2-oxo-2-[(oxoazepane-3-yl)amino]acetate, 3-benzoyl-1,2,3,10-b-tetrahydropyrrolo(2,1-A)isochinoline-1-carbonitrile, ajmalin, (2S,3R)-3-phenylpyrrolidine-2-carboxylic acid, 4-(2-hydroxyoctahydro-1,3,4-metheno-2H-cyclobuta(CD)pentalene-2-yl)phenol, methyl-C-4-(4-methoxyphenyl)-2-benzyl-7-phenyl-6,8-dioxo-3,7-diazabicyclo[3.3.0]-octane-R-2-carboxylate, methyl-C-4-(4-methoxyphenyl)-2-benzyl-7-(4-chlorophenyl)-6,8-dioxo-3,7-diazabicyclo[3.3.0]-octane-R-2-carboxylate, (+/-)-epibatidine dihydrochloride, (+)-epibatidin hydrochloride, 4-cyclopentyl-naphthalene-1-ylamine hydrochloride, 5-(2,3-dimethoxy-phenyl)-pyrazolidine-3-one, 5-(3,4-dimethoxy-phenyl)-pyrazolidine-3-one, 5-cyclopentyl-2-methoxy-4-nitrophenol, 1,3,5(10),6,8(beta)-estrapentaene-3-ol-17-oneacetate, norfluorocurarin, fluorocurarinchloride, 4,5-diphenylpyrazolidine-3-one, 4-(4-methoxyphenyl)-2-oxo-pyrrolidine-3-carbaxylic acid, 2-ethyl-2-(3-methoxyphenyl)pyrrolidine, N-(tert-butyl)-2-(isoxazole-5-ylcarbonyl)decahydroisochinoline-3-carboxamide, (+/-)-nicotin-D3-salicylate, benzyl-1,2-diphenyl-4-hydroxy-5-oxo-3-pyrroline-3-carboxylate, (1'S,2'S)-nicotin-1'-oxide, Specs CIF7952, 3-phenyl-(2-thienylmethyl)pyrrolidine-2,5-dione, N1-(4-[2,5-dioxo-1-[4-(trifluoromethoxy)phenyl]tetrahydro-1-H-pyrrole-3-yl]phenyl)-2,2,2-trifluoroacetamide and 1,3-diphenylcyclopentane-2,4,5-trione are fitted into the hydrophobic pocket as identified and are subsequently experimentally tested for their inhibition of the formation of the complex of β-catenin and its binding partners or its influence on the binding of cefamandol to β-catenin using ELISA, and subsequently are optimised by chemical modification.

4. Method according to any of claims 1 to 3, further comprising mixing of the therapeutic substance fitting into a pocket with a suitable pharmaceutical carrier.

5. Use of a method according to any of claims 1 to 4 in the context of the production of a pharmaceutical composition for the treatment of human diseases, tumours, such as colon carcinoma and melanoma, for tissue regeneration or for improving the growth of hair.

## Revendications

1. Procédé pour rechercher des substances qui empêchent sélectivement la liaison de la β-caténine sur les facteurs de transcription LEF-1/TCF, l'APC ou la conductine/axine, où
a) des logements hydrophobes sont identifiés dans la molécule β-caténine au voisinage des sites essentiels de liaison, et
b) ensuite, on synthétise et teste des substances thérapeutiques s'adaptant à ces logements.

2. Procédé selon la revendication 1, où
a) on identifie des mutants de la β-caténine, qui marquent les sites essentiels de liaison avec les facteurs de transcription LEF-1/TCF, l'APC ou la conductine,
b) on caractérise les surfaces de ces régions, sur base des données structurelles des cristaux par diffraction des rayons X du domaine Armadillo, et
c) on identifie ainsi les logements hydrophobes présents,
d) on ajuste des composés de faible poids moléculaire à ces logements et on les choisit sur base d'interactions stabilisantes avec la β-caténine et de l'inhibition ou de l'accélération sélective de la formation de complexe avec le LEF/TCF, l'APC ou la conductine dans des tests biologiques, et
e) ces composés, sont encore modifiés, le cas échéant par introduction de radicaux acides.

3. Procédé selon la revendication 1 ou 2, où
a) les mutants de la β-caténine Lys 435, Arg 469, His 470, Lys 508, Arg 515, qui marquent les sites essentiels de liaison avec le LEF-1,
b) les mutants de la β-caténine Phe 253, His 260, Lys 292, qui marquent les sites essentiels de liaison avec la conductine,
c) les mutants de la β-caténine Lys 345, Trp 383, Arg 386, qui marquent les sites essentiels de liaison avec l'APC, sont identifiés,
d) les surfaces des molécules sont caractérisées avec le programme Grasp, Spock, LUDI (MSI) ou autre, approprié pour le calcul de surfaces moléculaires,
e) on identifie un logement hydrophobe, entouré par les acides aminés Val 358, Met 363, Ala 391, Ala 392, Thr 393, Lys 394, Gln 395, Met 398, Leu 401, Leu 402, Ile 423, Asn 426, Leu 427, Thr 428, Cys 429, Asn 430, Asn 431, Asn 434, Met 437, Val 438, voisin du site de liaison avec le LEF-1/TCF, et
f) des composés, qui sont choisis parmi le groupe consistant en le céfamandol, le céfamandol-nafat, le sel du sodium du céfamandol, la céfsulodine, le cafadroxil, la céfatriaxone, l'AC-(6-O-stéaroyl)muramyl-Ala-D-isoglutamine, le 3,6-dihydroxybenzonorbornane, la 1-(4-fluorophényl)-3-phénylpyrrolidine-2,5-dione, la (-)-cotinine, le 4-(2-hydroxyoctahydro-1,3,4-méthéno-2H-cyclobuta(CD)pentalèn-2-yl)phénol, l'antho-ARNamide, le L-trans-époxusuccinyl-Leu-3-méthylbutylamide, le N-acétyl-muramyl-Ala-isoGln-OH, l'acétate de 1,3,5(10),6,8(14bêta)oestrapentaène-3-ol-17-one, le fumarate de (-)-oestrol, le N-acétyl-muramyl-Ala-D-isoGln-OH, le 2-oxo-2-[(oxoazépan-3-yl)amino]acétate d'éthyle, le 3-benzoyl-1,2,3,10-B-tétrahydropyrrolo-(2,1-a)isoquinoléine-1-carbonitrile, l'ajmaline, l'acide (2S,3R)-3-phénylpyrrolidine-2-carboxylique, le 4-(2-hydroxyoctahydro-1,3,4-méthéno-2H-cyclobuta(CD)-pentalèn-2-yl)phénol, le C-4-(4-méthoxyphényl)-2-benzyl-7-phényl-6,8-dioxo-3,7-diazabicyclo[3.3.0]-octane-R-2-carboxylate de méthyle, le C-4-(4-méthoxyphényl)-2-benzyl-7-(4-chlorophényl)-6,8-dioxo-3,7-diazabicyclo[3.3.0]octane-R-2-carboxylate de méthyle, le dichlorhydrate de la (+/-)-épibatidine, le chlorhydrate de la (+)-épibatidine, le chlorhydrate de la 4-cyclopentylnaphtalène-1-ylamine, la 5-(2,3-diméthoxyphényl)pyrazolidine-3-one, la 5-(3,4-diméthoxyphényl)pyrazolidine-3-one, le 5-cyclopentyl-2-méthoxy-4-nitrophénol, l'acétate de 1,3,5(10),6,8(bêta)-oestrapentaèn-3-ol-17-one, la norfluorcurarine, le chlorure de fluorcurarine, la 4,5-diphénylpyrazolidine-3-one, l'acide 4-(4-méthoxyphényl)-2-oxopyrrolidine-3-carboxylique, la 2-éthyl-2-(3-méthoxyphényl)pyrrolidine, le N-(t-butyl)-2-(isoxazol-5-ylcarbonyl)décahydroisoqionoléine-3-carboxamide, le D3-salicylate de (+/-)-nicotinyle, le 1,2-diphényl-4-hydroxy-5-oxo-3-pyrroline-3-carboxylate de benzyle, le (1'S,2'S)-nicotine-1'-oxyde, le CIF7952, la 3-phényl-(2-thiénylméthyl)pyrrolidine-2,5-dione, le N1-(4-[2, 5-dioxo-1-[4- (trifluorométhoxy)phényl]tétrahydro-1H-pyrrol-3-yl]phényl)-2,2,2-trifluoroacétamide, et la 1,3-diphénylcyclopentane-2,4,5-trione, sont ajustés dans les logements hydrophobes identifiés et ensuite, évalués expérimentalement par ELISA concernant leur inhibition de la formation de complexe avec la β-caténine et ses partenaires de liaison ou concernant l'influence de la liaison du céfamandol sur la β-caténine et ensuite, on optimalise par modification chimique.

4. Procédé selon l'une des revendications 1 à 3, comprenant d'autre part, le mélange d'une substance thérapeutique s'adaptant à un logement, avec un support pharmaceutique approprié.

5. Utilisation d'un procédé selon une des revendications 1 à 4, dans le cadre de la préparation d'une composition pharmaceutique pour le traitement de maladies humaines, de tumeurs comme le carcinome du colon et des mélanomes, pour la régénération tissulaire ou pour accélérer la pousse des cheveux.
